(19) Europäisches Patentamt
European Patent Office
Office européen des brevets

(11) Veröffentlichungsnummer: **0 248 335 B1**

# (12) EUROPÄISCHE PATENTSCHRIFT

(45) Veröffentlichungstag der Patentschrift: **31.10.90**

(51) Int. Cl.⁵: **C07D 239/26**, C09K 19/34

(21) Anmeldenummer: **87107658.4**

(22) Anmeldetag: **26.05.87**

(54) **Chirale Ester aus alpha-substituierten Carbonsäuren und mesogenen Pyrimidin-5-yl-phenolen und ihre Verwendung als Dotierstoff in Flüssigkristall-Phasen.**

(30) Priorität: **30.05.86 DE 3618213**
**11.09.86 DE 3630933**
**04.02.87 DE 3703228**

(43) Veröffentlichungstag der Anmeldung:
**09.12.87 Patentblatt 87/50**

(45) Bekanntmachung des Hinweises auf die Patenterteilung:
**31.10.90 Patentblatt 90/44**

(84) Benannte Vertragsstaaten:
**AT BE CH DE ES FR GB IT LI NL SE**

(56) Entgegenhaltungen:
**EP-A- 0 159 872**
**EP-A- 0 191 600**
**EP-A- 0 225 195**
**WO-A-86/06373**
**WO-A-87/05017**
**WO-A-87/05018**
**DD-A- 240 386**

(73) Patentinhaber: **HOECHST AKTIENGESELLSCHAFT, Postfach 80 03 20, D-6230 Frankfurt am Main 80(DE)**

(72) Erfinder: **Heppke, Gerd, Prof. Dr., Johann.Georg-Strasse 3, D-1000 Berlin 31(DE)**
Erfinder: **Bahr, Christian, Hohenzollerndamm 189, D-1000 Berlin 31(DE)**
Erfinder: **Müller, Ingrid, Dr., Am Pfingstbrunnen 1, D-6238 Hofheim am Taunus(DE)**
Erfinder: **Ohlendorf, Dieter, Dr., Am Kühlen Grund 4, D-6237 Liederbach(DE)**
Erfinder: **Wingen, Rainer, Dr., Rotkäppchenweg 10, D-6234 Hattersheim am Main(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Kennlinien der in Flüssigkristall-Displays verwendeten elektro-optischen Effekte verändern sich im allgemeinen mit der Temperatur. Insbesondere bei einer Ansteuerung im Multiplexbereich ergeben sich daraus Schwierigkeiten, die zu einer unerwünschten Einschränkung des Arbeitstemperaturbereiches führen können. Bei verschiedenen elektrooptischen Effekten kann durch Zusatz chiraler Verbindungen zum nematischen Flüssigkristall über die Temperaturfunktion der Ganghöhe der dadurch induzierten cholesterischen Helixstruktur die Temperaturabhägigkeit der elektrooptischen Kennlinien vorteilhaft beeinflußt werden, so beim cholesterisch-nematischen Phasenumwandlungseffekt, der TN ("twisted nematic")-Zelle und dem kürzlich vorgestellten SBE (supertwisted birefringence effect").

Neben den nematischen und cholesterischen Flüssigkristallen haben seit einigen Jahren in zunehmendem Maße auch für Praxisanwendungen geneigt ("tilted")-smektische Flüssigkristall-Phasen an Bedeutung gewonnen. Wenn solchen geneigt-smektischen Phasen, insbesondere smektisch C ($S_C$ oder SmC)Phasen, geeignete Dotierstoffe zugesetzt werden, die eine sogenannte spontane Polarisation ($P_S$) zeigen, so können die genannten Phasen in eine ferroelektrische Flüssigkristall-Phase umgewandelt werden (Benennung von $P_S$ in $nC \cdot cm^{-2}$), siehe dazu beispielsweise Lagerwall et al. im Aufsatz "Ferroelectric Liquid Cristals for Displays" (Ferroelektrische Flüssigkristalle für Anzeigenelemente), SID Symposium, October Meeting, 1985, San Diego (USA).

In der EP-A 0 159 872 werden Verbindungen der nachstehenden allgemeinen Formel beschrieben, die als Komponente in Flüssigkristall-Systemen geeignet sein sollen:

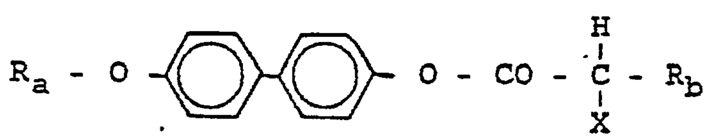

Die Substituenten sollen dabei folgende Bedeutung haben:

$R_a$ = ($C_1$–$C_{18}$)Alkyl, X = F, Cl oder Br und $R_b$ = verzweigtes ($C_3$–$C_7$)Alkyl, Benzyl oder Phenyl. Von der Verbindung mit $R_a$ = $C_8H_{17}$, X = Cl und $R_b$ = 1-Methyl-propyl ist ein Wert iür die spontane Polarisation von 210 $nC \cdot cm^{-2}$ angegeben.

Ferner werden in WO-A 8 606 373 neben einer Vielzahl von anderen Verbindungen auch Pyrimidin-2-yl-phenylester beschrieben, die als Komponente in flüssigkristallinen Mischungen eingesetzt werden können.

Die Synthese der Ester aus chiralen Carbonsäuren und Pyrimidin-2-yl-phenolen wird unter anderem in Beispiel 11 dieser Druckschrift beschrieben.

Aufgabe der vorliegenden Erfindung ist es, Verbindungen aufzuzeigen, die bei hohen Werten für die spontane Polarisation $P_S$ Strukturelemente aufweisen, die sie auch mit anderen Komponenten in Flüssigkristall-Systemen "verträglich" (d.h. mischbar) machen, da u.a. der mesogene Teil der Moleküle oftmals für eine gute "Verträglichkeit" mit den anderen Mischungskomponenten in Flüssigkristall-Systemen verantwortlich ist.

Darüber hinaus sollte ein breiter $S_C$*-Phasenbereich durch den Zusatz der erfindungsgemäßen Verbindungen bewirkt werden, um den für die praktische Anwendung nutzbaren Temperaturbereich der flüssigkristallinen Mischungen zu erweitern.

Die Bereitstellung von Verbindungen, die in einer flüssigkristallinen Mischung zu einem erhöhten Tiltwinkel – und damit zu einem erhöhten Kontrast im Display – führen ist ebenfalls Aufgabe der vorliegenden Erfindung.

Die Erfindung geht aus von den bekannten chiralen Estern aus α-substituierten Carbonsäuren und mesogenen Hydroxylverbindungen. Die erfindungsgemäßen Ester sind dann dadurch gekennzeichnet, daß in der allgemeinen Formel (I)

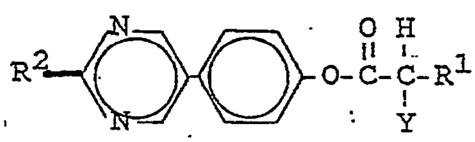

die Symbole folgende Bedeutung haben

Y = F, Cl, Br, CN oder $CF_3$, und

$R^1$ = verzweigtes ($C_3$–$C_9$)Alkyl,

Die genannten Verbindungen sind chirale α-substituierte (d.h. in 2-Stellung einer chiralen verzweigten Alkansäure substituierte) Alkansäureester von Pyrimidin-5-yl-phenolen.

Eine weitere Lösung der gestellten Aufgabe ist eine verdrillbare Flüssigkristall-Phase mit einem Gehalt an mindestens einer chiralen Verbindung, die als chirale Verbindung mindestens eine Verbindung der allgemeinen Formel (I) enthält. Unter dem Begriff "verdrillbare Flüssigkristall-Phase" sind nematische, cholesterische oder geneigt("tilted")-smektische, insbesondere SmC-Phasen zu verstehen.

Die verdrillbaren Flüssigkristall-Phasen bestehen aus 2 bis 20, vorzugsweise 2 bis 15 Komponenten, darunter mindestens einer der erfindungsgemäß beanspruchten chiralen Verbindungen. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe der chiralen Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt [= mindestens eine enantiotrope (Klärtemperatur > Schmelztemperatur) oder monotrope (Klärtemperatur < Schmelztemperatur) Mesophasenbildung erwarten läßt].

Insbesondere enthält die verdrillbare Flüssigkristall-Phase neben mindestens einer der erfindungsgemäß beanspruchten chiralen Verbindungen eine Phenylpyrimidinverbindung mit $S_C$-Phase, z.B. ein 4-(5-Alkyl-pyrimidin-2-yl)-1-alkoxybenzol, oder eine Ester-Verbindung mit $S_C$-Phase, z.B. einen 4-Alkyloxybenzoesäurephenylester.

Unter den Verbindungen der allgemeinen Formel (I) sind die bevorzugt, bei denen die Symbole folgende Bedeutung haben:

$R^2$ = geradkettiges oder verzweigtes ($C_4$–$C_{10}$)Alkyl oder Alkenyl, wobei eine $CH_2$-Gruppe durch ein O- oder S-Atom ersetzt sein kann, Y = Cl oder $CF_3$, $R^1$ = verzweigtes ($C_3$–$C_6$)Alkyl. Besonders bevorzugt sind Verbindungen der allgemeinen Formel (I) mit zwei chiralen Zentren im sich von der Carbonsäure ableitenden Rest, d.h. mit einem chiralen Zentrum am α-C-Atom und einem weiteren am Rest $R^1$.

Von der oder den erfindungsgemäßen Verbindung(en) enthalten die Flüssigkristall-Phasen im allgemeinen 0,01 bis 70 Gew.-%, insbesondere 0,05 bis 50 Gew.-%.

Die Herstellung der erfindungsgemäßen Verbindungen erfolgt zweckmäßigerweise durch Umsatz von mesogenen Verbindungen der Formeln (III) oder (III')

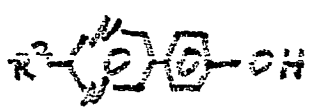

(III)

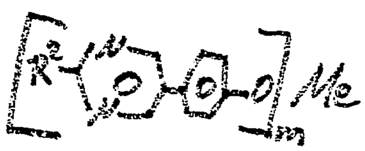

(III')

wobei Me für Erdalkalimetall oder vorzugsweise Alkalimetall steht und m = 1 (Alkalimetall) oder m = 2 (Erdalkalimetall) ist, mit Verbindungen der Formel (IV)

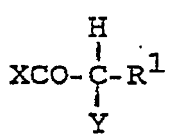

(IV)

in der X OH oder Halogen, vorzugsweise Chlor darstellt und Y sowie $R^1$ die obige Bedeutung haben.

Vorzugsweise werden für das erfindungsgemäße Herstellungsverfahren mesogene Hydroxylverbindungen (III) und Säurechloride (IV) (X = Cl) eingesetzt, wobei die Reaktion in Anwesenheit von Säurefängern, wie Aminen, beispielsweise Pyridin oder Triethylamin, oder von Alkali- oder Erdalkali(hydrogen)carbonaten, im allgemeinen bei Temperaturen von –40 bis +20°C erfolgt. Die Reinigung des erhaltenen Rohproduktes (I) kann in üblicher Weise, beispielsweise durch Säulenchromatographie und/oder Umkristallisation, geschehen.

Die erfindungsgemäßen Verbindungen sind insbesondere als Dotierstoffe für geneigt-smektische Flüssigkristall-Phasen geeignet, da sie diese in ferroelektrische Flüssigkristall-Phasen umwandeln; die Werte für die spontane Polarisation (Ps) liegen im Bereich von etwa 30 bis 250 nC · $cm^2$ (linear extrapoliert auf die reine Verbindung).

Beispiele

Herstellung der α-Halogencarbonsäure (Y = Cl)

Es werden 0,2 mol (S)-α-Aminosäure bei Raumtemperatur in 250 ml 6n-HCl gelöst. Dazu gibt man unter Rühren 0,3 mol $NaNO_2$ in kleinen Portionen, so daß die Temperatur unter 30°C bleibt. Nach beendeter Zugabe wird noch während 4 h bei Raumtemperatur nachgerührt. Es wird 3 mal mit Ether extrahiert, die Etherphase wird über $MgSO_4$ getrocknet und der Ether abdestilliert. Die Ausbeute an (S)-α-Chlorcarbonsäure beträgt 0,14 bis 0,16 mol. Entsprechend können auch die übrigen α-substituierten Carbonsäuren mit Y = F oder Br synthetisiert werden. Die Herstellung der entsprechenden Cyano- oder Trifluormethylverbindungen ist literaturbekannt, es wird auf Standardwerke verwiesen.

Herstellung des Säurechlorids

Es werden zu 0,2 mol (S)-α-substituierter Carbonsäure 60 ml $SOCl_2$ gegeben. Das Reaktionsgemisch wird während 3 h bei 50°C gerührt und danach das überschüssige $SOCl_2$ abdestilliert. Der Rückstand wird im Vakuum destilliert, wonach etwa 0,10 bis 0,18 mol des (S)-α-substituierten Carbonsäurechlorids erhalten werden.

Allgemeine Vorschrift für die Herstellung der Verbindungen (I)

Es werden 0,01 mol der Hydroxyverbindung in 50 ml Pyridin gelöst und dazu werden 0,01 mol des vorstehend beschriebenen Säurechlorids gegeben. Das Reaktionsgemisch wird während 1 h bei Raumtemperatur gerührt. Das ausgefallene Pyridinhydrochlorid wird abfiltriert und das Pyridin abdestilliert. Der Rückstand wird säulenchromatographisch (mit 6 Gew.-% $H_2O$ desaktiviertes Kieselgel, Laufmittel: Methylenchlorid) gereinigt und das Produkt drei- bis fünfmal aus Ethanol umkristallisiert, die Ausbeute beträgt 20 bis 60 %.

Vergleichsbeispiele V1 bis V6 (vergleiche auch WO-A 8 606 373)

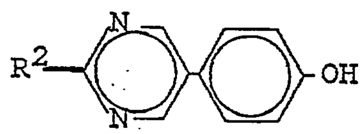

Die mesogene Hydroxylverbindung ist literaturbekannt (z.B. DE-C 2 257 588)

Tabelle 1

| Bsp.Nr. | $R^2$ | $R^1$ | Phasenfolge (°C) |
|---|---|---|---|
| V1 | $H_{19}C_9$ | $-CH(CH_3)_2$ | K 46 I<br>($S_C$30 $S_A$36 I) |
| V3 | $H_{15}C_7$ | $-CH_2CH(CH_3)_2$ | K 57 I |
| V4 | $H_{17}C_8$ | " | K 32 I |
| V5 | $H_{15}C_7$ | (S)-$CH(CH_3)C_2H_5$ | K 58 I |
| V6 | $H_{17}C_8$ | " | K 45 I |

Es bedeuten: K Kristallin, I isotrop, $S_A$, $S_B$, $S_C$, $S_I$: smektische A-, B-, C- oder I-Phasen

Werte in Klammern: Phasenfolge beim Abkühlen

Beispiele 1 bis 8

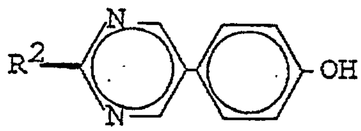

Die mesogene Hydroxylverbindung ist literaturbekannt [z.B. Heterocycles, Vol. 19, No. 6, 1079–1082 (1982)].

Tabelle 2

| Bsp.Nr. | $R^2$ | $R^1$ | Phasenfolge (°C) |
|---|---|---|---|
| 4 | $H_{17}C_8$ | $-CH_2CH(CH_3)_2$ | K 28 I<br>(K 19 $S_A$22 I) |
| 5 | $H_{23}C_{11}$ | " | K 49 I |
| 6 | $H_{17}C_8$ | (S)-$CH(CH_3)C_2H_5$ | K 50 I |
| 7 | $H_{19}C_9$ | " | K 52 I<br>(K 10 $S_A$26 I) |
| 8 | $H_{23}C_{11}$ | " | K 56 I<br>(K? $S_B$21 $S_A$29 I) |

Beispiele 9 bis 11

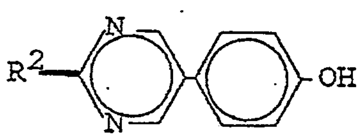

Die mesogene Hydroxylverbindung ist literaturbekannt (z.B. H. Zaschke, Diss. B. Univ. Halle, 1977).

| Besp.Nr. | $R^2$ | $R^1$ | Phasenfolge (°C) |
|---|---|---|---|
| 9 | $H_5C_2O$- | (S)-$CH(CH_3)C_2H_5$ | K 97 I |
| 10 | $H_{17}C_8O$- | " | K 66 I |
| 11 | $H_{19}C_9O$- | " | K 69 I<br>(K 35 $S_A$49 I) |

Beispiele 12 bis 17

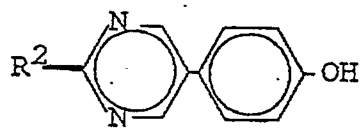

Die mesogene Hydroxylverbindung ist literaturbekannt [z.B. H. Zaschke, et al., Z. Chem. 17, 293 (1977)].

**Tabelle 4**

| Bsp.Nr. | $R^2$ | $R^1$ | Phasenfolge (°C) |
|---|---|---|---|
| 12 | $H_{15}C_7S$- | (S)-$CH(CH_3)C_2H_5$ | K 73 I |
| 14 | $H_{17}C_8S$- | " | K 65 I |
| 15 | $H_{19}C_9S$- | " | K 67 I |
| 16 | $H_{21}C_{10}S$- | " | K 63 I |
| 17 | $H_{23}C_{11}S$- | " | K 71 I |

Beispiele 18 bis 21

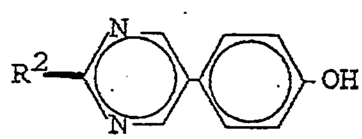

Bei Verwendung entsprechender Ausgangsmaterialien werden die mesogenen Hydroxylverbindungen wie bei den Beispielen 9 bis 11 bzw. 12 bis 17 erhalten.

Tabelle 5

| Bsp.Nr. | $R^2$ | $R^1$ | Phasenfolge (°C) |
|---|---|---|---|
| 18 | (S)-$H_5C_2CH(CH_3)CH_2O$- | (S)-$CH(CH_3)C_2H_5$ | K 83 I |
| 19' | (S)-$(H_3C)_2C{=}CHCH_2CH(CH_3)CH_2CH_2O$- | " | K 44 I |
| 20 | $H_2C{=}CH$-$(CH_2)_9O$- | " | K 74 I |
| 21 | (S)-$H_5C_2CH(CH_3)(CH_2)_5S$- | " | K 82 I |

Anwendungsbeispiele

Zur Überprüfung der Wirksamkeit der vorstehend beschriebenen Verbindungen als Dotierstoffe in Flüssigkristall-Systemen werden diese in Konzentrationen von jeweils 10 Mol-% (oder in einzelnen Fällen von 17,5 und 25 Mol-%) mit dem Racemat der Verbindung

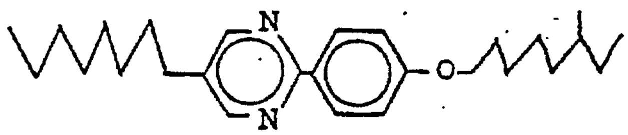

A

Phasenfolge: K 14,9°C $S_c$ 49,8°C $S_A$ 59,2°C I (5°C)
4-(5-Octyl-pyrimidin-2-yl)-1-(6-methyl-oct-1-oxy)benzol bzw. der Verbindung

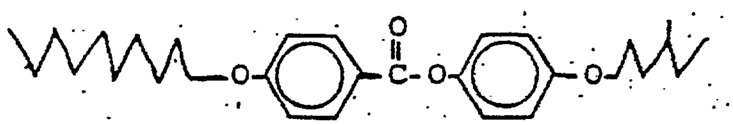

B

Phasenfolge: K 17°C $S_G$ 32,7°C $S_c$ 70,4°C $S_A$ 73,3°C I (–3°C)

4-(4-Decyloxy-phenyl-1-carbonyloxy)-1-(4-methyl-hexyl-oxy)-benzol gemischt und jeweils die Werte für die spontane Polarisation ($P_s$ in nC · cm–2), für die Schaltzeit $\tau$ (in ms) und für den optischen Neigungswinkel der $S_c$-Phase $\theta$ (in°) der Mischung bestimmt. Die $P_s$-Werte werden nach der Methode von H. Diamant et al. (Rev. Sci. Instr., 28, 30, 1957) gemessen, wobei eine spezielle Meßzelle [Skarp et al. in Ferroelectric Letters Vol. 06, 000 (1986)] verwendet wird, in der auch die $\tau$- und $\theta$-Werte bestimmt werden. Bei einer Zellenschichtdicke von ca. 2 µm wird durch Scherung eine einheitliche planare Orientierung der Flüssigkristalle in der $S_c$-Phase erreicht [SSFC-Technik, Clark et al., Appl Phys. Lett. 36, 899 (1980)]. Zur Bestimmung von $\tau$ und $\theta$ wird die Meßzelle auf dem Drehtisch eines Polarisationsmikroskops zwischen gekreuztem Analysator und Polarisator befestigt. Durch Drehen der Meßzelle von maximalem zu minimalem Lichtdurchgang wird der optische Neigungswinkel bzw. Schaltwinkel, 2 $\theta$, bestimmt. Mit Hilfe einer Photodiode erfolgt die Bestimmung der Schaltzeit, $\tau$, indem die Anstiegszeit des Lichtsignals von 10 auf 90% Signalhöhe gemessen wird. Die Tabellen fassen die Ergebnisse für die Mischungen zusammen. Neben den Werten für $P_s$, $\tau$, 2$\theta$ ist der $S_c$-Bereich der jeweiligen Mischung angegeben; die Werte in Klammern geben dabei die unterkühlbare untere Temperaturgrenze des $S_c$-Bereichs an. Bei Verwendung der Verbindung A (B) als Wirt zur Aufnahme des Dotierstoffes beziehen sich alle Werte für $P_s$, $\tau$ und 2$\theta$ auf eine Temperatur von 25°C (40°C).

In der Tabelle 6 sind einige Ergebnisse für die chiralen Vergleichs-Verbindungen des Typs

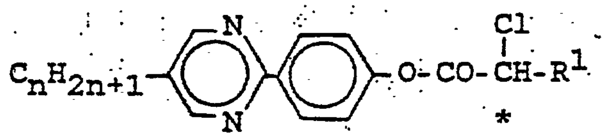

zusammengefaßt, die ausschließlich als Dotierstoffe in der Verbindung A untersucht wurden.

Tabelle 6

| Verbindung Bsp.Nr. | Anteil d.Verbindung in mol% | $S_c$*-Bereich der Mischung in °C | $P_s$ | τ | 2θ |
|---|---|---|---|---|---|
| V4 | 10 | 10 bis 28 (3) | 0,2 | 15 | 8 |
| V5 | 10 | 10 bis 33 (0) | 3,5 | 55 | 21 |
| V6 | 10 | 7 bis 37 (3) | 6,5 | 27 | 27 |

Tabelle 7 enthält Ergebnisse für die erfindungsgemäßen Verbindungen

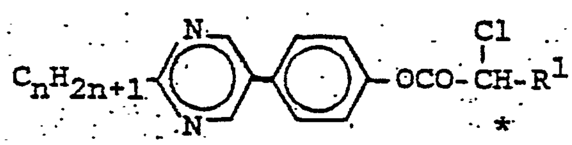

bei Verwendung als Dotierstoffe in der Verbindung A. Wie ein Vergleich zwischen den Tabellen 6 und 7 und insbesondere der Vergleich zwischen den Verbindungen V4 und 4, sowie V6 und 6, zeigt, verbessert die Umkehrung der Pyrimidingruppe das Mischungsverhalten der genannten Verbindungen, d.h. der $S_C$-Bereich der Wirtsverbindung A wird durch Zugabe der Verbindungen 4 und 6 weniger eingeschränkt als durch Zugabe der Vergleichsverbindungen V4 und V6.

Tabelle 10

| Verbindung Bsp.Nr. | Anteil d.Verbindung in mol% | $S_c$*-Bereich der Mischung in °C | $P_s$ | τ | 2θ |
|---|---|---|---|---|---|
| 4 | 10 | 22 bis 46 (10) | 7,5 | 150 | 45 |
| 4 | 25 | 34 bis 39 (15) | 19 | 17 | 37 |
| 6 | 10 | 15 bis 46 (0) | 13 | 48 | 42 |
| 6 | 25 | 27 bis 42 (16) | 38 | 20 | 47 |
| 7 | 10 | 6 bis 49 (0) | 14 | 48 | 48 |
| 7 | 17,5 | 13 bis 45 (2) | 25 | 20 | 49 |
| 7 | 25 | 21 bis 42 (6) | 32 | 12 | 50 |
| 8 | 10 | 16 bis 49 (3) | 14 | 38 | 44 |
| 8 | 17,5 | 23 bis 47 (6) | 23 | 35 | 50 |
| 8 | 25 | 38 bis 46 (10) | 36 | 30 | 50 |

Tabelle 8 faßt einige Ergebnisse für Mischungen der chiralen Verbindungen

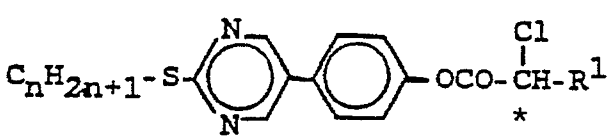

mit der Verbindung A zusammen. Wie ein Vergleich zwischen den Tabellen 7, 8 und 9 und insbesondere zwischen den Verbindungen V6, 6 und 14, sowie 8 und 17 zeigt, wird durch die Einführung der Thioether-Brücke nochmals das Mischungsverhalten der genannten Dotierstoffe verbessert. Überraschenderweise vergrößern die Verbindungen 14 und 21 bei einem Zusatz von 10 mol% den $S_C$-Bereich der Wirtsverbindung A. Diese Eigenschaft ist für die praktische Anwendung der genannten Dotierstoffe, bei der möglichst große $S_C$-Bereiche gefordert werden, von großem Vorteil.

## Tabelle 11

| Verbindung Bsp.Nr. | Anteil d.Verbindung in mol% | Wirt | $S_c$*-Bereich der Mischung in °C | $P_s$ | τ | 2θ |
|---|---|---|---|---|---|---|
| 12 | 10 | A | 15(1) bis 46 | 10,7 | 37 | 48,5 |
| 14 ~~20~~ | 10 | A | 6,7(1) bis 49,5 | nicht untersucht | | |
| ~~23~~ | 10 | A | 18,7(1) bis 49,5 | 12,5 | 30 | 48 |
| ~~23~~ | 25 | A | 29,4(15) bis 41 | 35 | 20 | 52 |
| ~~27~~ | 10 | A | 0(0) bis 47 | 14 | 48 | 51 |

Tabelle 9 faßt einige Ergebnisse für Mischungen der chiralen Verbindungen

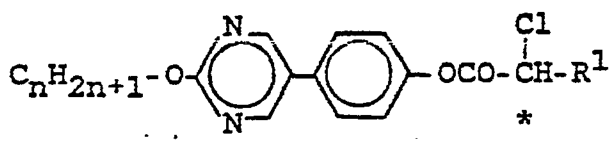

mit der Verbindung A und B zusammen. Der Vergleich zwischen den Tabellen 7 und 8 und insbesondere zwischen den Verbindungen V6, 6 und 10, sowie 7 und 10 zeigt, daß durch die Einführung der Ether-Brücke ebenfalls das Mischungsverhalten der genannten Dotierstoffe verbessert wird. Überraschenderweise führt eine Dotierung der Verbindungen A und B mit 10 mol% der Verbindungen 10 und 11 zu einer Vergrößerung der $S_C$-Bereiche der beiden Verbindungen A und B.

## Tabelle 9

| Verbindung Bsp.Nr. | Anteil d.Verbindung in mol% | Wirt | $S_c$*-Bereich der Mischung in °C | $P_s$ | τ | 2θ |
|---|---|---|---|---|---|---|
| 10 | 10 | A | 3,7(0) bis 49,5 | 14 | 40 | 51 |
| 10 | 25 | A | 17,3(-5) bis 49,5 | 38,5 | 25 | 57 |
| 11 | 10 | A | 4,0(0) bis 52 | 13 | 60 | 48 |
| 11 | 10 | B | 20,6(20) bis 62,8 | 7 | 70 | 53 |
| 19 | 10 | A | 10,4(0) bis 38 | 13 | 55 | 51 |
| 20 | 10 | A | 4,2(-2) bis 38 | 13 | 62 | 50 |

**Patentansprüche für die Vertragsstaaten: BE, CH, DE, FR, GB, IT, LI, NL, SE**

1. Chiraler Ester aus α-substituierten Carbonsäuren und mesogenen Hydroxylverbindungen gemäß der allgemeinen Formel (I),

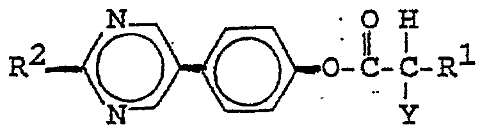

(I)

wobei die Symbole folgende Bedeutung haben:

$R^2$ = geradkettiges oder verzweigtes ($C_1$–$C_{12}$)Alkyl oder Alkenyl, wobei eine oder zwei nichtbenachbarte $CH_2$-Gruppen durch O- und/oder S-Atome ersetzt sein können,

$R^1$ = verzweigtes ($C_3$–$C_9$)Alkyl, und

Y = F, Cl, Br, CN oder $CF_3$.

2. Chiraler Ester nach Anspruch 1, dadurch gekennzeichnet, daß in der allgemeinen Formel (I) die Symbole folgende Bedeutung haben:

$R^2$ = geradkettiges ($C_4$–$C_{10}$)Alkyl oder Alkenyl, wobei eine $CH_2$-Gruppe durch ein O- oder S-Atom ersetzt sein kann

$R^1$ = verzweigtes ($C_3$–$C_6$)Alkyl und

Y = Cl oder $CF_3$.

3. Chirale Ester nach Anspruch 1 oder 2, dadurch gekennzeichnet, daß in der allgemeinen Formel (I) der Rest $R^1$ ein weiteres chirales Zentrum aufweist.

4. Verdrillbare Flüssigkristall-Phase mit einem Gehalt an mindestens einer chiralen Verbindung der allgemeinen Formel (I) nach Anspruch 1.

5. Flüssigkristall-Anzeigeelement enthaltend eine Flüssigkristall-Phase nach Anspruch 4.

6. Verwendung einer chiralen Verbindung gemäß der allgemeinen Formel (I) nach Anspruch 1 zur Umwandlung einer geneigt-smektischen in eine ferroelektrische Flüssigkristall-Phase.

7. Verfahren zur Umwandlung einer geneigt-smektischen in eine ferroelektrische Flüssigkristall-Phase durch Zusatz von mindestens einer chiralen Verbindung, dadurch gekennzeichnet, daß man der Flüssigkristall-Phase mindestens eine Verbindung der allgemeinen Formel (I) nach Anspruch 1 zusetzt.

**Patentansprüche für die Vertragsstaaten: AT, ES**

1. Verfahren zur Herstellung chiraler Ester aus α-substituierten Carbonsäuren und mesogenen Hydroxylverbindungen gemäß der allgemeinen Formel (I),

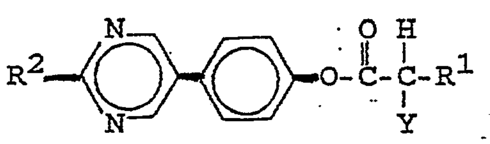

(I)

wobei die Symbole folgende Bedeutung haben:

$R^2$ = geradkettiges oder verzweigtes ($C_1$–$C_{12}$)Alkyl oder Alkenyl, wobei eine oder zwei nichtbenachbarte $CH_2$-Gruppen durch O- und/oder S-Atome ersetzt sein können,

$R^1$ = verzweigtes ($C_3$–$C_9$)Alkyl, und

Y = F, Cl, Br, CN oder $CF_3$,

dadurch gekennzeichnet, daß man eine Hydroxyverbindung der allgemeinen Formel (III)

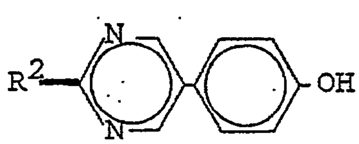

(III)

bzw. ein Alkali- oder Erdalkalisalz dieser Verbindung mit einer Verbindung der Formel (IV) umsetzt,

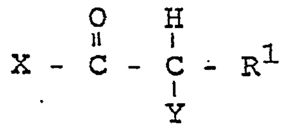 (IV)

wobei X = OH oder Halogen ist und Y und $R^1$ die obige Bedeutung haben.

2. Verfahren nach Anspruch 1, dadurch gekennzeichnet, daß in der allgemeinen Formel (I) die Symbole folgende Bedeutung haben:

$R^2$ = geradkettiges $(C_4–C_{10})$Alkyl oder Alkenyl, wobei eine $CH_2$-Gruppe durch ein O- oder S-Atom ersetzt sein kann

$R^1$ = verzweigtes $(C_3–C_6)$Alkyl

X = OH oder Halogen und

Y = Cl oder $CF_3$.

3. Verfahren nach Anspruch 1 oder 2, dadurch gekennzeichnet, daß in der allgemeinen Formel (I) der Rest $R^1$ ein weiteres chirales Zentrum aufweist.

4. Verdrillbare Flüssigkristall-Phase mit einem Gehalt an mindestens einer chiralen Verbindung der allgemeinen Formel (I) nach Anspruch 1.

5. Flüssigkristall-Anzeigeelement enthaltend eine Flüssigkristall-Phase nach Anspruch 4.

6. Verwendung einer chiralen Verbindung gemäß der in Anspruch 1 aufgeführten allgemeinen Formel (I) zur Umwandlung einer geneigt-smektischen in eine ferroelektrische Flüssigkristall-Phase.

7. Verfahren zur Umwandlung einer geneigt-smektischen in eine ferroelektrische Flüssigkristall-Phase durch Zusatz von mindestens einer chiralen Verbindung, dadurch gekennzeichnet, daß man der Flüssigkristall-Phase mindestens eine Verbindung der in Anspruch 1 aufgeführten allgemeinen Formel (I) zusetzt.

**Claims for the Contracting States: BE, CH, DE, FR, GB, IT, LI, NL, SE**

1. A chiral ester formed from an a-substituted carboxylic acid and a mesogenic hydroxyl compound of the general formula (I)

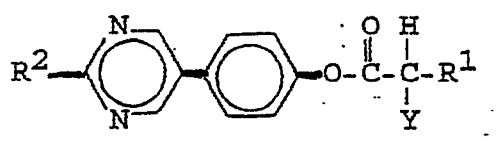 (I)

in which the symbols have the following meaning:

$R^2$ is a straight-chain or branched $(C_1–C_{12})$alkyl or alkenyl, it being possible for one or two non adjacent $CH_2$ groups to be replaced by O and/or S atoms,

$R^1$ is branched $(C_3–C_9)$alkyl, and

Y is F, Cl, Br, CN or $CF_3$.

2. A chiral ester as claimed in claim 1, wherein the symbols in the general formula (I) have the following meaning:

$R^2$ = straight-chain $(C_4–C_{10})$alkyl or alkenyl, it being possible for a $CH_2$ group to be replaced by an 0 or S atom,

$R^1$ = branched $(C_3–C_6)$alkyl, and

Y = Cl or $CF_3$.

3. A chiral ester as claimed in claim 1 or 2, wherein the radical $R^1$ in the general formula (I) has a further chiral center.

4. A twistable liquid-crystalline phase with a content of at least one chiral compound of the general formula (I) as claimed in claim 1.

5. A liquid-crystalline display element containing a liquid-crystalline phase as claimed in claim 4.

6. The use of a chiral compound of the general formula (I) as claimed in claim 1 for transforming a tilted smectic liquid-crystalline phase into a ferroelectric liquid-crystalline phase.

7. A method for converting a tilted smectic liquid-crystalline phase into a ferroelectric liquid-crystalline phase by adding at least one chiral compound, wherein at least one compound of the general formula (I) as claimed in claim 1 is added to the liquid-crystalline phase.

**Claims for the Contracting States: AT, ES**

1. A process for the preparation of a chiral ester fosned from an α-substituted carboxylic acid and a mesogenic hydroxyl compound of the general formula (I)

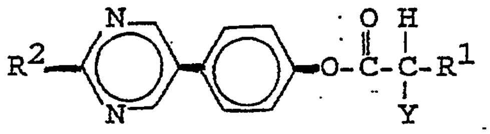
(I)

in which the symbols have the following meaning:

$R^2$ is a straight-chain or branched $(C_1–C_{12})$alkyl or alkenyl, it being possible for one or two nonadjacent $CH_2$ groups to be replaced by O and/or S atoms,

$R^1$ is branched $(C_3–C_9)$alkyl, and

Y is F, Cl, Br, CN or $CF_3$,

which comprises reacting a hydroxyl compound of the general formula (III)

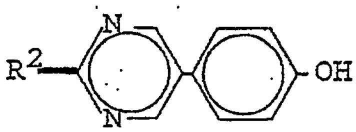
(III)

or an alkali metal or alkaline earth metal salt of said compound with a compound of the formula (IV)

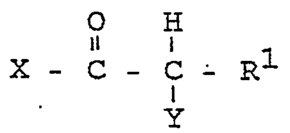
(IV)

in which X = OH or halogen and Y and $R^1$ have the above meaning.

2. The process as claimed in claim 1, wherein the symbols in the general formula (I) have the following meaning:

$R^2$ = straight-chain $(C_4–C_{10})$alkyl or alkenyl, it being possible for a $CH_2$ group to be replaced by an O or S atom,

$R^1$ = branched $(C_3–C_6)$alkyl,

X = OH or halogen and

Y = Cl or $CF_3$.

3. The process as claimed in claim 1 or 2, wherein the radical $R^1$ in the general formula (I) has a further chiral center.

4. A twistable liquid-crystalline phase with a content of at least one chiral compound of the general formula (I) as claimed in claim 1.

5. A liquid-crystalline display element containing a liquid-crystalline phase as claimed in claim 4.

6. The use of a chiral compound of the general formula (I) indicated in claim 1 for transforming a tilted smectic liquid-crystalline phase into a ferroelectric liquid-crystalline phase.

7. A method for converting a tilted smectic liquid-crystalline phase into a ferroelectric liquid-crystalline phase by adding at least one chiral compound, wherein at least one compound of the general formula (I) indicated in claim 1 is added to the liquid-crystalline phase.

**Revendications pour les Etats contractants: BE, CH, DE, FR, GB, IT, LI, NL, SE**

1. Ester chiral d'acides carboxyliques α-substitués et de composés hydroxylés mésogènes selon la formule générale (I)

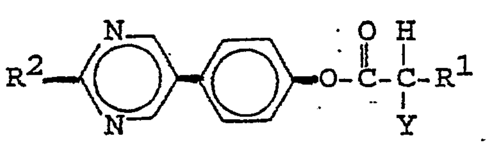
(I)

où les symboles ont les significations suivantes:

$R^2$ est un radical alkyle ou alcényle en $C_1$ à $C_{12}$ à chaîne droite ou ramifiée, un ou deux groupes $CH_2$ non voisins pouvant être remplacés par des atomes O et/ou S,

$R^1$ est un radical alkyle en $C_3$ à $C_9$ à chaîne ramifiée, et

Y = F, Cl, Br, CN ou $CF_3$.

2. Ester chiral selon la revendication 1, caractérisé en ce que, dans la formule générale (I), les symboles ont les significations suivantes:

$R^2$ est un radical alkyle ou alcényle en $C_4$ à $C_{10}$ à chaîne droite, un groupe $CH_2$ pouvant être remplacé par un atome O ou S, $R^1$ est un radical alkyle en $C_3$–$C_6$ ramifié et Y est Cl ou $CF_3$.

3. Esters chiraux selon la revendication 1 ou 2, caractérisés en ce que, dans la formule générale (I), le radical $R^1$ présente un centre chiral supplémentaire.

4. Phase mésomorphe torsadable contenant au moins un composé chiral de formule générale (I) selon la revendication 1.

5. Elément d'affichage à cristaux liquides contenant une phase mésomorphe selon la revendication 4.

6. Utilisation d'un composé chiral selon la formule générale (I) de la revendication 1 pour convertir une phase mésomorphe smectique-inclinée en une phase mésomorphe ferro-électrique.

7. Procédé pour convertir une phase mésomorphe smectique-inclinée en une phase mésomorphe ferro-électrique par addition d'au moins un composé chiral, caractérisé en ce qu'on ajoute à la phase mésomorphe au moins un composé de formule générale (I) selon la revendication 1.

**Revendications pour les Etats contractants: AT, ES**

1. Procédé de préparation d'esters chiraux d'acides carboxyliques α-substitués et de composés hydroxylés mésogènes selon la formule générale (I)

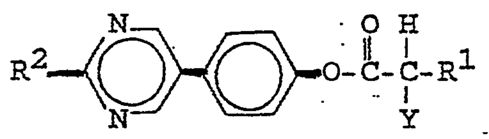

(I)

dans laquelle les symboles ont les significations suivantes:

$R^2$ est un radical alkyle ou alcényle en $C_1$ à $C_{12}$ à chaîne droite ou ramifiée, un ou deux groupes $CH_2$ non voisins pouvant être remplacés par des atomes O et/ou S,

$R^1$ est un radical alkyle en $C_3$ à $C_9$ à chaîne ramifiée, et

Y = F, Cl, Br, CN ou $CF_3$,

caractérisé en ce qu'on fait réagir un composé hydroxylé de formule générale (III)

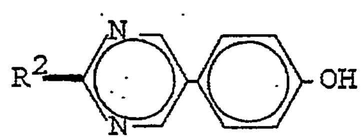

(III)

ou un sel de métal alcalin ou de métal alcalino-terreux de ce composé, avec un composé de formule (IV)

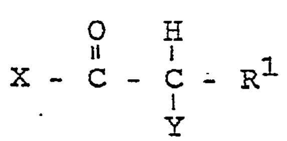

(IV)

dans laquelle X = OH ou un halogène, et Y et $R^1$ ont les significations données ci-dessus.

2. Procédé selon la revendication 1, caractérisé en ce que, dans la formule générale (I), les symboles ont les significations suivantes:

$R^2$ est un radical alkyle ou alcényle en $C_4$–$C_{10}$ à chaîne droite, un groupe $CH_2$ pouvant être remplacé par un atome O ou S,

$R^1$ est un radical alkyle en $C_3$–$C_6$ ramifié,

X est OH ou un halogène, et

Y est Cl ou $CF_3$.

3. Procédé selon la revendication 1 ou 2, caractérisé en ce que, dans la formule générale (I), le radical $R^1$ présente un centre chiral supplémentaire.

4. Phase mésomorphe torsadable contenant au moins un composé chiral de formule générale (I) selon la revendication 1.

5. Elément d'affichage à cristaux liquides contenant une phase mésomorphe selon la revendication 4.

6. Utilisation d'un composé chiral selon la formule générale (I) donnée dans la revendication 1 pour convertir une phase mésomorphe smectique-inclinée en une phase mésomorphe ferro-électrique.

7. Procédé pour convertir une phase mésomorphe smectique-inclinée en une phase mésomorphe ferro-électrique par addition d'au moins un composé chiral, caractérisé en ce qu'on ajoute à la phase mésomorphe au moins un composé de formule générale (I) selon la revendication 1.